# EUROPEAN PATENT SPECIFICATION

(11) **EP 3 479 869 B1**
(45) Date of publication and mention of the grant of the patent: **02.06.2021**
(21) Application number: 17851944.3
(22) Date of filing: 18.10.2017
(51) Int. Cl.: A61N 1/04

(54) **MICRO-CURRENT WOUND DRESSING AND CONTROL METHOD THEREFOR**
MIKROSTROMWUNDVERBAND UND STEUERUNGSVERFAHREN DAFÜR
PANSEMENT À MICRO-COURANT ET SON PROCÉDÉ DE COMMANDE

(30) Priority: 07.09.2017 CN 201710801506
(43) Date of publication of application: 08.05.2019
(73) Proprietor: Henan Huibo Medical Co., Ltd., Nanyang, Henan 473000 (CN)
(72) Inventor: WANG, Chuan, Nanyang Henan 473000 (CN); YAN, Meng, Nanyang Henan 473000 (CN); ZHOU, Xinqin, Nanyang Henan 473000 (CN); ZHANG, Jianxin, Nanyang Henan 473000 (CN)
(74) Representative: Lusuardi, Werther
(86) International application number: PCT/CN2017/106756
(87) International publication number: WO 2019/047335

(56) References cited:
- WO-A1-2014/178945
- WO-A1-2016/142401
- CN-A- 104 284 695
- CN-A- 105 797 197
- US-A- 5 395 398

## Description

### Technical Field

The present disclosure relates to the technical field of medical supplies, and particularly to a micro-current wound-protecting dressing and a control method thereof. WO2014178945A1 discloses expandable wound dressings; US5395398A discloses a microelectric apparatus for the antisepsis, promulgation of healing and analgesia of wound and chronic skin ulcers; WO2016142401A1 discloses a method of forming a medical device comprising grapheme; CN104284695A discloses a skin dressing having electrodes and physiologically active precursor substances; and CN105797197A discloses a skin dressing.

### Background Art

As the integrity of skin and mucosa is destroyed, wounds caused by various injuries lead to local pain, bleeding and dysfunction. If concurrent infection occurs, pain is exacerbated, accompanied with inflammatory reactions, i.e., congestion, swelling and exudation. If fat is liquefied, exudation increases; and granulation tissues poorly grows, and healing is slow. Therefore, wounds should be treated in a proper way, so that the wounds can be prevented from being infected and inflamed, and healing of the wounds can be promoted. Relevant researches have been done on the application of micro-current to the treatment of various wounds, and the effects of micro-current are mainly reflected in: reducing inflammation reactions of the wounds, promoting blood vessel regeneration, and accelerating epithelization of the wounds.

In most of the existing micro-current wound-protecting dressings, zinc, silver and other active electrodes are placed on the lower surface of the dressing to form a primary battery structure to be self-powered. Such dressings achieve miniaturization and integration. However, due to the characteristics of the active electrodes, the power supply of such a micro-battery structure is unstable, and the electrodes can be easily inactivated in the conductive environment; in addition, the materials of the active electrodes are generally metals such as silver and zinc as well as oxides thereof, and when the electrodes are brought into direct contact with a wound, they will injure the wound cells. Moreover, the active electrodes gradually wear down in use due to the oxidation-reduction reaction, will gradually be inactivated as the time of using the dressing lapses, and will stop generating current. The rate of wearing down of the active electrodes determines the service life of the dressing, which usually shortens the service life of the dressing.

### Disclosure of the Invention

The invention is defined in the appended claims. Aspects, embodiments and examples disclosed herein which do not fall within the scope of the appended claims do not form part of the invention, and are merely provided for illustrative purposes.

In order to overcome the defects in the prior art, one of the objects of the present disclosure is to provide a micro-current wound-protecting dressing capable of generating stable current on a wound surface. Moreover, the electrodes thereof can be made of a polymer material having the electricity conducting function and good biocompatibility, which prevents injuries to the wound cells; moreover, the entire dressing is compact in structure and is convenient to use.

In order to achieve the above object, the present disclosure adopts the following technical solution:
a micro-current wound-protecting dressing, comprising a dressing base body, a power supply module provided on the dressing base body, and at least two first electrodes and at least two second electrodes that are arranged in parallel, opposite to each other and spaced from each other on the dressing base body, materials used for the first electrodes and the second electrodes being biocompatible conductive polymer materials, wherein different first electrodes are connected with each other in parallel and are connected with a positive electrode of the power supply module; different second electrodes are connected with each other in parallel and connected with a negative electrode of the power supply module; and every two adjacent electrodes are different electrodes.

The biocompatible conductive polymer material refers to polymer materials that have good biocompatibility and can conduct electricity among the existing commercially available materials, for example, a conductive rubber material with a good biocompatibility.

Optionally, the power supply module is provided with a control circuit for controlling the power supply module to supply power and stop supplying power, and controlling the magnitude of a voltage output of the power supply module.

The control circuit achieves voltage regulation by regulating the input frequency of an inductance booster circuit using the principle of digital MCU simulation converter in the main circuit. The voltage of the power supply can be regulated to 0-1000 V, and then the voltage is output in the form of a high-speed short pulse by the control circuit. The duration of the high-speed short pulse voltage signal can be controlled according to the specific use environment. Optionally, the control circuit is configured to control the frequency of the voltage output of the power supply module and the duration of a single output. Further, the duration of the single output is 0.1-10 µs. Further, the duration of the single output is 0.5-2 µs. Further, the output energy per time is 0.0005-0.005 W. For example, the duration of the high-speed short pulse voltage can be controlled to be 1 µs, so that the output energy is only about 0.001 W per time, or even smaller, which ensures the safety of the product when it is applied to human body. In addition, controlled switching between starting power supply and stopping power supply is realized by connecting the switches in series in the main circuit.

In the using process, when the wound surface does not need any current stimulation, the power supply can be cut off by the control circuit so as to stop power supply of the power supply module; or the magnitude of the output voltage of the power supply module can be regulated according to the specific conditions of the wound surface, so as to control the magnitude of the current applied to the wound surface, ensuring the stability and controllability of the current stimulation to the wound surface.

Optionally, the magnitude of the current generated in the wound-protecting dressing is 0-100 mA. Optionally, the magnitude of the current of the micro-current wound-protecting dressing flowing through the wound surface is 0-100 mA; optionally, the magnitude of the current of the micro-current wound-protecting dressing flowing through the wound surface is 1-90 mA; optionally, the magnitude of the current of the micro-current wound-protecting dressing flowing through the wound surface is 10-80 mA; and optionally, the magnitude of the current of the micro-current wound-protecting dressing flowing through the wound surface is 1-8 mA.

Optionally, in order to ensure that the dressing is compact in structure, small in size and convenient to carry and use, the power supply of the power supply module is a direct-current power supply, and further, the power supply body of the power supply module is a micro-battery, such as a button battery.

Optionally, with the side close to the wound surface as an inner layer, the dressing base body is a one-piece structure, and comprises a backing layer and a skin-friendly layer which has a wound therapeutic effect sequentially from exterior to interior.

Optionally, the material used for the skin-friendly layer is a conductive material, the first electrode and the second electrode are embedded in the skin-friendly layer, and the skin-friendly layer wraps the first electrode and the second electrode. Further, the skin-friendly layer comprises a first conductive portion and a second conductive portion that are insulated from each other, and the first electrode and the second electrode are situated at the first conductive portion and the second conductive portion, respectively. Since the first electrode and the second electrode are wrapped in the skin-friendly layer and the skin-friendly layer is made of a conductive material, such as a conductive gel, when the power supply module is powered on, with the skin-friendly layer as a conducting medium, a loop is formed between the adjacent first electrode and second electrode to generate current, the current is conducted to the wound surface through the skin-friendly layer and acts on the wound surface, producing adjuvant therapeutic effect and healing promoting effect on the wound surface. Similarly, when the materials used for the backing layer and the skin-friendly layer are conductive materials, the first electrode and the second electrode can also be arranged on the backing layer to generate current with the backing layer as a conducting medium, which current is then conducted to the wound surface through the skin-friendly layer and acts on the wound surface.

Optionally, the first electrode and the second electrode are arranged on the skin-friendly layer, and the first electrode and/or the second electrode protrude(s) from the surface (inner surface) of the skin-friendly layer that is used for contacting the wound surface. That is, the case can be that both the first electrode and the second electrode protrude from the surface of the skin-friendly layer that is used for contacting the wound surface, or can be that one of the first electrode and the second electrode protrudes from the surface of the skin-friendly layer that is used for contacting the wound surface, and the other is substantially flush with the surface of the skin-friendly layer that is used for contacting the wound surface. When the first electrode and/or the second electrode are/is arranged to protrude from the inner surface of the skin-friendly layer, the skin-friendly layer may not use a conductive material, in this case, since the first electrode and the second electrode are in direct contact with the wound surface, the exudate of the wound surface, the disinfectant or so forth on the wound surface can be used as a conducting medium, when the power supply module begins to supply power, a loop is formed between the adjacent first electrode and second electrode to generate current, and the current directly acts on the wound surface, producing adjuvant therapeutic effect and healing promoting effect on the wound surface.

In order to ensure that the first electrode and the second electrode form an effective loop after the power supply module supplies power to cause the dressing to produce an effective current intensity. Further, preferably, the distance between adjacent electrodes is 0.5-2 mm. Optionally, the distance between adjacent electrodes is 0.8-1.2 mm. Optionally, the distance between adjacent electrodes is 1 mm.

Besides the one-piece structure, the dressing base body can also have a split structure. Optionally, the dressing base body comprises a first dressing base body and a second dressing base body that are arranged in parallel and apart from each other, the first electrode is arranged on the first dressing base body, and the second electrode is arranged on the second dressing base body; and the materials used for the first dressing base body and the second dressing base body are conductive materials.

When in use, the first dressing base body and the second dressing base body are respectively adhered to the skin on the two opposite sides of the wound surface, after the power supply module supplies power, with the first dressing base body, the second dressing base body and the wound surface jointly as a conducting medium, the first electrode and the second electrode form a loop and generate current to act on the wound surface.

Optionally, the first dressing base body and the second dressing base body have an identical structure, and with the side close to the wound surface as the inner layer, both the first dressing base body and the second dressing base body each comprise a backing layer and a skin-friendly layer which has a wound therapeutic effect from exterior to interior; and the materials used for the backing layer and the skin-friendly layer are conductive materials, and the first electrode and the second electrode are embedded in the backing layer or the skin-friendly layer.

Optionally, the first dressing base body and the second dressing base body have an identical structure, and with the side close to the wound surface as the inner layer, the first dressing base body and the second dressing base body each comprise a backing layer and a skin-friendly layer which has a wound therapeutic effect from exterior to interior; and the material used for the skin-friendly layer is a conductive material, and the first electrode and the second electrode are embedded in the skin-friendly layer.

The first electrode and the second electrode may have a shape of rectangle, square, circle, oval, polygon, etc.; preferably, the first electrode and the second electrode are rectangular and have a strip look.

Optionally, the size of the backing layer is larger than that of the skin-friendly layer, and the portion of the backing layer beyond the skin-friendly layer is provided circumferentially with an adhesive layer. In other words, when the micro-current wound-protecting dressing is laid on a plane, the orthographic projection of the skin-friendly layer on the plane is smaller than the orthographic projection of the backing layer on the plane, and further, the orthographic projection of the skin-friendly layer on the plane is completely within the orthographic projection of the backing layer on the plane.

When the material used for the skin-friendly layer is a self-adhesive gel material, the size of the backing layer can be set to be the same as that of the skin-friendly layer, the adhesive layer is omitted, and the dressing base body is adhered to the wound surface or to the skin in the vicinity of the wound surface through the self-adhesive property of the gel material.

In order to protect the skin-friendly layer of the dressing and at the same time prevent the wound surface from being infected, the material used for the backing layer is a waterproof breathable material, such as a polyurethane film. The material used for the skin-friendly layer with the wound therapeutic effect can be a gel material, and in order to endow the dressing with a good wound therapeutic effect, the gel material contains pharmaceutical ingredients such as antibacterial and healing-promoting ingredients.

The present disclosure further provides a micro-current wound-protecting dressing, comprising a dressing base body, a power supply module provided on the dressing base body, and a wire, with the side close to the wound surface as an inner layer, the dressing base body comprising a backing layer and a skin-friendly layer which has a wound therapeutic effect from exterior to interior; and the material used for the skin-friendly layer being is a conductive material, and the skin-friendly layer being electrically connected with the power supply module through the wire.

The material used for the skin-friendly layer is a conductive gel with self-adhesive property.

The present disclosure further provides a method for controlling any of the above micro-current wound-protecting dressings, which method comprises outputting a voltage in the form of a high-speed short pulse through a control circuit.

Further, the duration of the high-speed short pulse voltage is controlled to be 0.1-10 µs. Further, the duration of the high-speed short pulse voltage is controlled to be 0.5-2 µs. Further, the duration of the high-speed short pulse voltage is controlled to be 1 µs. The high-speed short pulse voltage ranges from 0 V to 1000 V. Optionally, the high-speed short pulse voltage ranges from 1 V to 900 V. Optionally, the high-speed short pulse voltage ranges from 5 V to 30 V. Optionally, the high-speed short pulse voltage ranges from 100 V to 800 V.

The micro-current wound-protecting dressing of the present disclosure characterized in that a power supply module is provided on the dressing base body, the electrodes are made of a polymer material with an electricity conducting function, no oxidation-reduction reactions occur during the utilization of the dressing, the electrodes do not wear down and have good stability, and the electrical conductivity is not reduced in some special environments, which prolongs the service life of the entire dressing. Moreover, compared with existing active electrodes made of metal oxides, the electrodes in the present disclosure utilize biocompatible conductive polymer materials, have good biocompatibility and are harmless to human body.

In general, the micro-current wound-protecting dressing of the present disclosure is portable and convenient to use, can generate stable and controllable current and has a long service life; moreover, the materials of the electrodes are harmless to human body.

### Brief Description of Drawings

FIG. 1 is a schematic structural diagram of a micro-current wound-protecting dressing provided by embodiment 1 of the present disclosure at a first visual angle;
FIG. 2 is a schematic structural diagram of the micro-current wound-protecting dressing provided by embodiment 1 of the present disclosure at a second visual angle;
FIG. 3 is a schematic structural diagram of a micro-current wound-protecting dressing provided by embodiment 2 of the present disclosure at a first visual angle;
FIG. 4 is a schematic structural diagram of the micro-current wound-protecting dressing provided by embodiment 2 of the present disclosure at a second visual angle;
FIG. 5 is a schematic structural diagram of a micro-current wound-protecting dressing provided by embodiment 3 of the present disclosure at a first visual angle;
FIG. 6 is a schematic structural diagram of the micro-current wound-protecting dressing provided by embodiment 3 of the present disclosure at a second visual angle;
FIG. 7 is a schematic structural diagram of a micro-current wound-protecting dressing provided by embodiment 4 of the present disclosure at a first visual angle;
FIG. 8 is a schematic structural diagram of the micro-current wound-protecting dressing provided by embodiment 4 of the present disclosure at a second visual angle;
FIG. 9 is a schematic structural diagram of a micro-current wound-protecting dressing provided by embodiment 5 of the present disclosure at a first visual angle; and

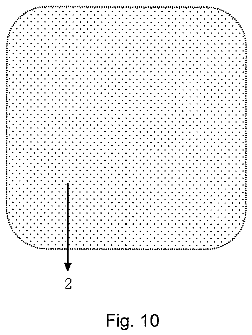

FIG. 10 is a schematic structural diagram of the micro-current wound-protecting dressing provided by embodiment 5 of the present disclosure at a second visual angle.

Reference signs: 1-backing layer; 2-skin-friendly layer; 3-first electrode; 4-second electrode; 5-power supply module; 6-adhesive layer; and 7-wire.

### Detailed Description of Embodiments

The biocompatible conductive polymer material can be a composite conductive polymer material (prepared using a general polymer material and various conductive substances, such as graphite, metal powder, metal fibers, metal oxides, carbon black and carbon fibers, through different methods and processing technologies, such as dispersion polymerization, filling and compounding, laminating and compounding, forming a surface electrolemma, or so forth), or can be a structural conductive polymer material (a polymer structure *per se* or a polymer material doped to have electricity conducting function). The biocompatible conductive polymer material in the following embodiments refers to conductive polymer materials with good biocompatibility among the existing commercially available materials, such as conductive rubber materials with good biocompatibility (such as silicone rubber with metal particles dispersed therein).

### Embodiment 1

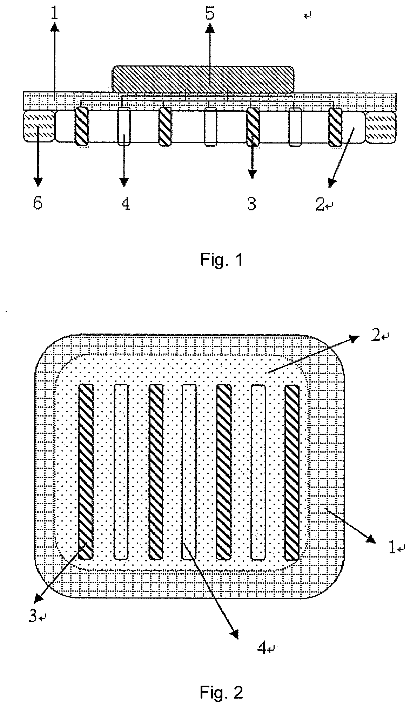

A micro-current wound-protecting dressing, as shown in FIG. 1 and FIG. 2, comprises a one-piece dressing base body, a power supply module 5 provided on the dressing base body, and a first electrode 3 and a second electrode 4 that are arranged in parallel, opposite to each other and apart from each other on the dressing base body; the first electrode 3 is connected with the positive electrode of the power supply module, and the second electrode 4 is connected with the negative electrode of the power supply module 5; the materials used for the first electrode 3 and the second electrode 4 are biocompatible conductive polymer materials; the first electrode 3 is in the number of four, and the second electrode is in the number of three, every two adjacent electrodes are different electrodes, the distance between adjacent electrodes is 0.5-2 mm, that is, the distance between the adjacent first electrode and second electrode is 0.5-2 mm, and further, the distance between adjacent first electrode and second electrode is 0.8-1.2 mm. Further, the distance between adjacent first electrode and second electrode is 1 mm.

The one-piece dressing base body comprises a backing layer 1 and a skin-friendly layer 2, the first electrodes 3 and the second electrodes 4 are arranged on the skin-friendly layer 2, and the inner surfaces thereof protrude from the inner surface of the skin-friendly layer 2 by 0-0.5 mm, and further, the first electrodes 3 and the second electrodes 4 protrude from the inner surface of the skin-friendly layer 2 by 0-0.5 mm. Further, the first electrodes 3 and the second electrodes 4 protrude from the inner surface of the skin-friendly layer 2 by 0-0.1 mm. Further, the first electrodes 3 and the second electrodes 4 protrude from the inner surface of the skin-friendly layer 2 by 0.01-0.1 mm. The material used for the skin-friendly layer 2 is a water absorbing and moisturizing gel material, when the skin-friendly layer is a non-conductive material or does not have a high electrical conductivity, after the dressing is applied to the wound surface, the first electrodes 3 and the second electrodes 4 are in direct contact with the wound surface, the exudate of the wound surface, the disinfectant or so forth on the wound surface can be used as a conducting medium, and when the power supply module begins to supply power, a loop is formed between adjacent first electrodes 3 and second electrodes 4 to generate current, the current directly acts on the wound surface, producing adjuvant therapeutic effect and healing promoting effect on the wound surface.

In other embodiments, when the skin-friendly layer 2 is made of a conductive material, the skin-friendly layer 2 comprises a first conductive portion and a second conductive portion that are insulated from each other, the number of the first conductive portion and of the second conductive portion is corresponding to the number of the electrodes, for example, if a pair of positive and negative electrodes are provided, then there are correspondingly provided one first conductive portion and one second conductive portion, and if two pairs of positive and negative electrodes are provided, then there are provided two first conductive portions and two second conductive portions. The first electrode 3 and the second electrode 4 are embedded in the skin-friendly layer, and are situated at the first conductive portion and the second conductive portion, respectively, and the skin-friendly layer wraps the first electrode 3 and the second electrode 4. When the skin-friendly layer 2 is not in contact with the wound surface, the first electrode 3 and the second electrode 4 are insulated from each other, when the skin-friendly layer 2 is in contact with the wound surface, the first conductive portion and the second conductive portion of the skin-friendly layer 2, and the exudate of the wound surface, the disinfectant on the wound surface and so forth are used as a conducting medium, and after the power supply module 5 begins to supply power, current flowing through the wound surface can be formed between the first electrode 3 and the second electrode 4. In one embodiment, the skin-friendly layer 2 made of a conductive material can be partitioned into two portions using an insulating material, and the two portions can be used as the first conductive portion and the second conductive portion, respectively. In addition, in the present embodiment, the portion of the skin-friendly layer 2 wrapping one of the electrodes and the portion of the skin-friendly layer 2 wrapping the other electrode should be insulated from each other, or at least have a resistance greater than the wound surface.

In order to adhere and fix the dressing to the wound surface more effectively, in the present embodiment, the size of the backing layer 1 is larger than that of the skin-friendly layer 2, and the lower surface of the portion of the backing layer 1 beyond the skin-friendly layer 2 is provided circumferentially with an adhesive layer 6.

In the present embodiment, the power supply module 5 is provided with a control circuit; the control circuit is used for controlling the power supply module 5 to supply power and stop supplying power, and controlling the magnitude of a voltage output of the power supply module. The control circuit achieves voltage regulation by regulating the input frequency of an inductance booster circuit using the principle of digital MCU simulation converter in the main circuit. The voltage of the power supply can be regulated to 0-1000 V, and then the voltage is output in the form of a high-speed short pulse by the control circuit. The duration of the high-speed short pulse voltage signal can be controlled according to the specific use environment. For example, the duration of the high-speed short pulse voltage can be controlled to be 1 µs, so that the output energy is only about 0.001 W per time, or even smaller, which ensures the safety of the product when it is applied to human body. In addition, controlled switching between starting power supply and stopping power supply is realized by connecting the switches in series in the main circuit. The magnitude of the current generated in the wound-protecting dressing is 0-100 mA.

In the using process, when the wound surface does not need any current stimulation, the power supply can be cut off by the control circuit so as to stop power supply of the power supply module 5; or the magnitude of the output voltage of the power supply module can be regulated according to the specific conditions of the wound surface, so as to control the magnitude of the current applied to the wound surface, ensuring the stability and controllability of the current stimulation to the wound surface.

In order to ensure that the dressing is compact in structure, small in size and convenient to carry and use, the power supply body of the power supply module in the present embodiment is a micro-battery, such as a button battery.

In the present embodiment, the first electrode 3 and the second electrode 4 have a shape of rectangle with a strip look, which can more effectively ensure the stability and the uniform coverage of the current. However, in the present embodiment, the first electrode and the second electrode can have a shape of square, circle, oval, polygon, etc.

In order to protect the skin-friendly layer 2 of the dressing and at the same time prevent the wound surface from being infected, the material used for the backing layer 1 in the present embodiment is a waterproof breathable material, such as a polyurethane film. The material used for the skin-friendly layer 2 with the wound therapeutic effect in the present embodiment can be a gel material, and in order to endow the dressing with a good wound therapeutic effect, the gel material contains pharmaceutical ingredients such as antibacterial and healing-promoting ingredients.

### Embodiment 2

The implementation principle of the micro-current wound-protecting dressing provided by the present embodiment is substantially the same as that in embodiment 1, and for the sake of brief description, as to the aspects not mentioned in this embodiment, reference can be made to the corresponding contents in embodiment 1.

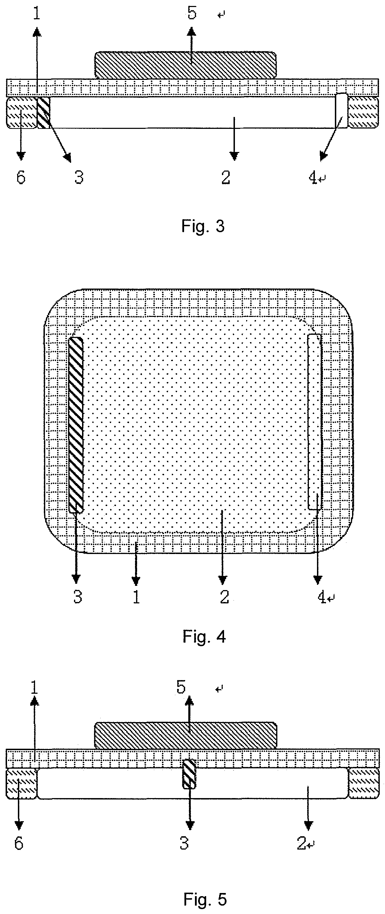

The differences between the micro-current wound-protecting dressing of the present embodiment, as shown in FIG. 3 and FIG. 4, and that in embodiment 1 lie in that in the dressing of the present embodiment, the first electrode 3 is in the number of one and the second electrode 4 is in the number of one, and the first electrode 3 and the second electrode 4 are arranged at the left end and the right end of the skin-friendly layer 2 of the dressing base body, respectively, the material used for the skin-friendly layer 2 is a conductive gel, the first electrode 3 and the second electrode 4 are embedded in the skin-friendly layer 2, and the skin-friendly layer 2 wraps the first electrode 3 and the second electrode 4.

In the present embodiment, the first electrode 3 and the second electrode 4 are wrapped in the skin-friendly layer, the skin-friendly layer 2 is made of a conductive material, when the power supply module 5 is powered on, with the skin-friendly layer 2 as a conducting medium, a loop is formed between the first electrode 3 and the second electrode 4 to generate current, the current is conducted to the wound surface through the skin-friendly layer 2 and acts on the wound surface, producing adjuvant therapeutic effect and healing promoting effect on the wound surface.

Similarly, when the backing layer 1 and the skin-friendly layer 2 are both made of conductive materials, the first electrode 3 and the second electrode 4 can also be arranged on the backing layer 1, and generate current with the backing layer 1 as a conducting medium, and the current is conducted to the wound surface through the skin-friendly layer 2 and acts on the wound surface. When the backing layer 1 and the skin-friendly layer 2 are both made of conductive materials, similar to the case where only the skin-friendly layer 2 is made of a conductive material, the backing layer 1 needs to be partitioned into at least two portions (the specific number is corresponding to the number of the electrodes, and if a pair of positive and negative electrodes are used, then the backing layer 1 is partitioned into two portions) that are insulated from each other, the backing layer 1 and each portion of the skin-friendly layer 2 are not directly electrified, but rather electrically conducted through the wound surface (as to the external loop, the current sequentially flows through the first electrode 3, the backing layer 1, the skin-friendly layer 2, the wound surface, the skin-friendly layer 2, the backing layer 1 and the first electrode 3), to generate current flowing through the wound surface.

### Embodiment 3

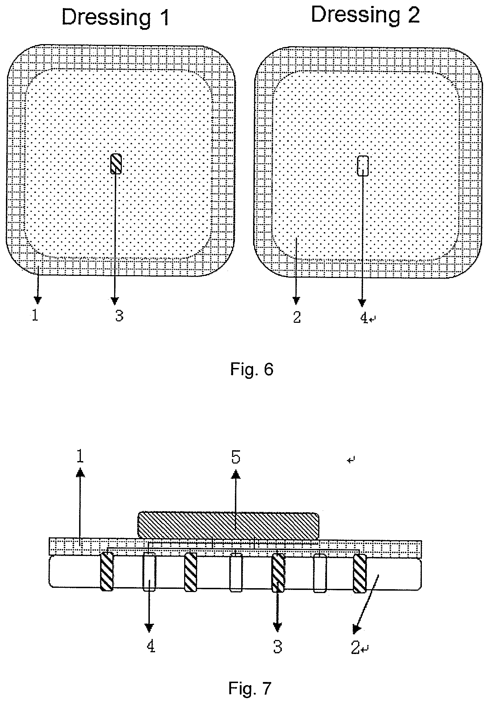

The micro-current wound-protecting dressing in this embodiment, as shown in FIG. 5 and FIG. 6, differs from that in embodiment 2 in that the dressing base body has a split structure and comprises a first dressing base body and a second dressing base body that have the same structure as the dressing base body in embodiment 2. The first electrode 3 is embedded in the skin-friendly layer 2 of the first dressing base body, and the second electrode 4 is embedded in the skin-friendly layer 2 of the second dressing base body in; further, the material used for the skin-friendly layer 2 is a conductive material. In the present embodiment, the first dressing base body and the second dressing base body are separated from each other, and during use, a gap is reserved between the first dressing base body and the second dressing base body, the first dressing base body and the second dressing base body are each equivalent to one electrode. For example, when in use, the first dressing base body and the second dressing base body are respectively adhered to the skin on the two opposite sides of the wound surface, after the power supply module 5 supplies power, with the skin-friendly layer 2 and the wound surface jointly as a conducting medium, the first electrode 3 and the second electrode 4 form a loop to generate current which acts on the wound surface.

Likewise, reference can be made to embodiment 2, when the backing layer 1 and the skin-friendly layer 2 are both made of conductive materials, the first electrode 3 and the second electrode 4 can also be arranged on the backing layer to form a loop with the backing layer 1, the skin-friendly layer 2 and the wound surface jointly as a conducting medium, to generate current which acts on the wound surface.

### Embodiment 4

The implementation principle of the micro-current wound-protecting dressing provided by the present embodiment is substantially the same as that in embodiment 1, and for the sake of brief description, as to the aspects not mentioned in this embodiment, reference can be made to the corresponding contents in embodiment 1.

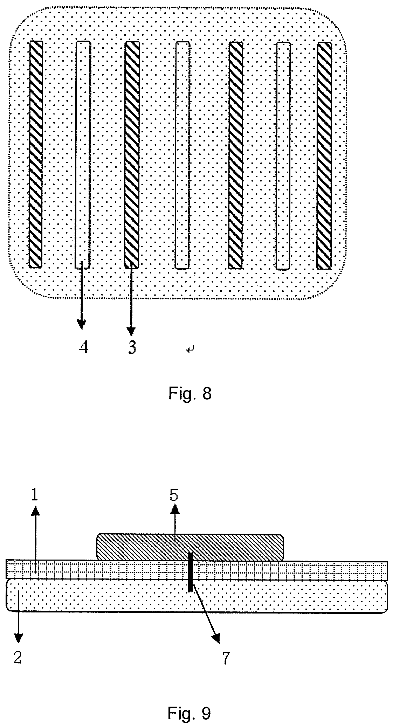

The micro-current wound-protecting dressing of the present embodiment, as shown in FIG. 7 and FIG. 8, differs from that in embodiment 1 in that the material used for the skin-friendly layer 2 is a gel material with self-adhesive property, and the adhesive layer is omitted. In the present embodiment, the size of the backing layer 1 is substantially the same as the size of the skin-friendly layer 2, and the edge of the backing layer 1 substantially coincides with that of the skin-friendly layer 2. In other words, when the micro-current wound-protecting dressing is laid on a plane, the orthographic projection of the skin-friendly layer 2 on the plane is substantially equal to the orthographic projection of the backing layer 1 on the plane. When the material used for the skin-friendly layer 2 is a gel material with self-adhesive property, in other embodiments, the backing layer 1 can have a different size from the skin-friendly layer 2.

### Embodiment 5

The implementation principle of the micro-current wound-protecting dressing provided by the present embodiment is substantially the same as that in embodiment 2, and for the sake of brief description, as to the aspects not mentioned in this embodiment, reference can be made to the corresponding contents in embodiment 2.

Different from embodiment 2, the micro-current skin-protecting dressing disclosed by the present embodiment is not provided with an electrode, the power supply module 5 and the skin-friendly layer 2 are connected through a wire 7, and the material of the skin-friendly layer 2 is a conductive gel with self-adhesive property. After the power supply module 5 is powered on, with the conductive gel and the human body as the conducting medium, current flows through the control circuit, the conductive gel, the wound surface and the human body, and is finally conducted to the ground. The electric stimulation of the current to the wound surface can produce adjuvant therapeutic effect and healing promoting effect.

The implementation modes of the present disclosure have been described in detail above with the embodiments with reference to the drawings.

### Industrial Applicability

The micro-current wound-protecting dressing of the present disclosure is portable and convenient to use, can generate stable and controllable current, and has a long service life, and the materials of the electrodes are harmless to the human body; therefore, the micro-current wound-protecting dressing is suitable for industrial production.

## Claims

1. A micro-current wound-protecting dressing, comprising a dressing base body, a power supply module (5) provided on the dressing base body, and at least two first electrodes (3) and at least two second electrodes (4) that are arranged in parallel, opposite to each other and spaced from each other on the dressing base body, materials used for the at least two first electrodes (3) and the at least two second electrodes (4) being biocompatible conductive polymer materials, **characterized in that** different first electrodes (3) are connected with each other in parallel and are connected with a positive electrode of the power supply module (5); different second electrodes (4) are connected with each other in parallel and connected with a negative electrode of the power supply module (5); every two adjacent electrodes are different electrodes; the dressing base body, with a side close to a wound surface as an inner layer, is in a one-piece structure and comprises, sequentially from exterior to interior, a backing layer (1) and a skin-friendly layer (2) which has a wound therapeutic effect.

2. The micro-current wound-protecting dressing according to claim 1, wherein the power supply module (5) is provided with a control circuit, and the control circuit is configured to control the power supply module (5) to supply power and stop supplying power, and configured to control a voltage output of the power supply module (5).

3. The micro-current wound-protecting dressing according to claim 2, wherein the control circuit is configured to control a frequency of the voltage output of the power supply module (5) and a duration for a single output.

4. The micro-current wound-protecting dressing according to claim 1, wherein a material used for skin-friendly layer (2) is a conductive material, the skin-friendly layer (2) comprises a first conductive portion and a second conductive portion that are insulated from each other, the first electrodes (3) and the second electrodes (4) are embedded in the skin-friendly layer (2) and are located at the first conductive portion and the second conductive portion respectively, and the skin-friendly layer (2) wraps the first electrodes (3) and the second electrodes (4).

5. The micro-current wound-protecting dressing according to claim 1 or 4, wherein the first electrodes (3) and the second electrodes (4) are arranged on the skin-friendly layer (2), and the first electrodes (3) and/or the second electrodes (4) protrude out from a surface of the skin-friendly layer (2), with the surface being used for contacting the wound surface.

6. The micro-current wound-protecting dressing according to any one of claims 1, 4 or 5, wherein materials used for the backing layer (1) and the skin-friendly layer (2) are both conductive materials, and the first electrodes (3) and the second electrodes (4) are arranged on the backing layer (1).

7. The micro-current wound-protecting dressing according to any one of claims 1 or 4-6, wherein a size of the backing layer (1) is larger than a size of the skin-friendly layer (2), and a portion of the backing layer (1) beyond the skin-friendly layer (2) is provided circumferentially with an adhesive layer (6).

8. The micro-current wound-protecting dressing according to any one of claims 1 or 4-6, wherein the skin-friendly layer (2) is made of a gel material with self-adhesive property.

## Patentansprüche

1. Mikrostromwundschutzverband, umfassend einen Verbandgrundkörper, ein Stromzufuhrmodul (5), das an dem Verbrandgrundkörper bereitgestellt ist, und zumindest zwei erste Elektroden (3) und zumindest zwei zweite Elektroden (4), die parallel, gegenüber einander und voneinander beabstandet an dem Verbandgrundkörper angeordnet sind, wobei es sich bei Materialien, die für die zumindest zwei ersten Elektroden (3) und die zumindest zwei zweiten Elektroden (4) verwendet werden, um biokompatible leitende Polymermaterialien handelt, **dadurch gekennzeichnet, dass** verschiedene erste Elektroden (3) zueinander parallel geschaltet und mit einer positiven Elektrode des Leistungszufuhrmoduls (5) verbunden sind; verschiedene zweite Elektroden (4) zueinander parallel geschaltet und mit einer negativen Elektrode des Leistungszufuhrmoduls (5) geschaltet sind; es sich bei allen zwei benachbarten Elektroden um unterschiedliche Elektroden handelt; der Verbandgrundkörper, der eine Seite aufweist, die als eine Innenschicht nahe an einer Wundfläche liegt, eine einstückige Struktur darstellt und sequentiell vom Außenbereich zum Innenbereich eine Stützschicht (1) und eine hautverträgliche Schicht (2) umfasst, die eine wundtherapeutische Wirkung aufweist.

2. Mikrostromwundschutzverband nach Anspruch 1, wobei das Stromzufuhrmodul (5) mit einer Steuerungsschaltung bereitgestellt ist und die Steuerungsschaltung konfiguriert ist, um das Stromversorgungsmodul (5) zu steuern, um Strom zuzuführen und die Stromzufuhr zu unterbrechen, und konfiguriert ist, um eine Spannungsausgabe des Stromzufuhrmoduls (5) zu steuern.

3. Mikrostromwundschutzverband nach Anspruch 2, wobei die Steuerungsschaltung konfiguriert ist, um eine Frequenz der Spannungsausgabe des Stromzufuhrmoduls (5) und eine Dauer einer einzigen Ausgabe zu steuern.

4. Mikrostromwundschutzverband nach Anspruch 1, wobei es sich bei einem Material, das für die hautverträgliche Schicht (2) verwendet wird, um ein leitendes Material handelt, die hautverträgliche Schicht (2) einen ersten leitenden Abschnitt und einen zweiten leitenden Abschnitt umfasst, die voneinander isoliert sind, die ersten Elektroden (3) und die zweiten Elektroden (4) in der hautverträglichen Schicht (2) eingebettet sind und sich jeweils in dem ersten leitenden Abschnitt und dem zweiten leitenden Abschnitt befinden und die hautverträgliche Schicht (2) die ersten Elektroden (3) und die zweiten Elektroden (4) umhüllt.

5. Mikrostromwundschutzverband nach Anspruch 1 oder 4, wobei die ersten Elektroden (3) und die zweiten Elektroden (4) auf der hautverträglichen Schicht (2) angeordnet sind und die ersten Elektroden (3) und/oder die zweiten Elektroden (4) von einer Fläche der hautverträglichen Schicht (2) hervorstehen, wobei die Fläche zum Berühren der Wundfläche verwendet wird.

6. Mikrostromwundschutzverband nach einem der Ansprüche 1, 4 oder 5, wobei es sich bei beiden der Materialien, die für die Stützschicht (1) und die hautverträgliche Schicht (2) verwendet werden, um leitende Materialien handelt und die ersten Elektroden (3) und die zweiten Elektroden (4) auf der Stützschicht (1) angeordnet sind.

7. Mikrostromwundschutzverband nach einem der Ansprüche 1 oder 4-6, wobei eine Größe der Stützschicht (1) größer ist als eine Größe der hautverträglichen Schicht (2) und ein Abschnitt der Stützschicht (1) über die hautverträgliche Schicht (2) hinaus in Umfangsrichtung mit einer Klebeschicht (6) bereitgestellt ist.

8. Mikrostromwundschutzverband nach einem der Ansprüche 1 oder 4-6, wobei die hautverträgliche Schicht (2) aus einem Gelmaterial mit selbstklebender Eigenschaft hergestellt ist.

## Revendications

1. Pansement de protection de plaie à micro-courant, comprenant un corps de base de pansement, un module d'alimentation de puissance (5) prévu sur le corps de base de pansement, et au moins deux premières électrodes (3) et au moins deux secondes électrodes (4) qui sont agencées en parallèle, opposées les unes aux autres et espacées les unes des autres sur le corps de base de pansement, des matériaux utilisés pour les au moins deux premières électrodes (3) et les au moins deux secondes électrodes (4) étant des matériaux de polymères conducteurs biocompatibles, **caractérisé en ce que** des premières électrodes (3) différentes sont connectées les unes aux autres en parallèle et sont connectées à une électrode positive du module d'alimentation de puissance (5) ; des secondes électrodes (4) différentes sont connectées les unes aux autres en parallèle et connectées à une électrode négative du module d'alimentation de puissance (5) ; toutes les paires d'électrodes adjacentes sont des électrodes différentes ; le corps de base de pansement, avec un côté proche d'une surface de plaie en tant que couche interne, est une structure à une pièce et comprend, séquentiellement de l'extérieur à l'intérieur, une couche de renfort (1) et une couche adaptée à la peau (2) qui a un effet thérapeutique sur une plaie.

2. Pansement de protection de plaie à micro-courant selon la revendication 1, dans lequel le module d'alimentation de puissance (5) est muni d'un circuit de commande, et le circuit de commande est configuré pour commander le module d'alimentation de puissance (5) pour une alimentation de puissance et un arrêt d'alimentation de puissance, et configuré pour commander une sortie de tension du module d'alimentation de puissance (5).

3. Pansement de protection de plaie à micro-courant selon la revendication 2, dans lequel le circuit de commande est configuré pour commander une fréquence de la sortie de tension du module d'alimentation de puissance (5) et une durée pour une seule sortie.

4. Pansement de protection de plaie à micro-courant selon la revendication 1, dans lequel un matériau utilisé pour la couche adaptée à la peau (2) est un matériau conducteur, la couche adaptée à la peau (2) comprend une première portion conductrice et une seconde portion conductrice qui sont isolées l'une de l'autre, les premières électrodes (3) et les secondes électrodes (4) sont incorporées dans la couche adaptée à la peau (2) et sont situées au niveau de la première portion conductrice et de la seconde portion conductrice respectivement, et la couche adaptée à la peau (2) enveloppe les premières électrodes (3) et les secondes électrodes (4).

5. Pansement de protection de plaie à micro-courant selon la revendication 1 ou 4, dans lequel les premières électrodes (3) et les secondes électrodes (4) sont agencées sur la couche adaptée à la peau (2), et les premières électrodes (3) et/ou les secondes électrodes (4) font saillie à partir d'une surface de la couche adaptée à la peau (2), la surface étant utilisée pour entrer en contact avec la surface de plaie.

6. Pansement de protection de plaie à micro-courant selon l'une quelconque des revendications 1, 4 ou 5, dans lequel des matériaux utilisés pour la couche de renfort (1) et la couche adaptée à la peau (2) sont tous deux des matériaux conducteurs, et les premières électrodes (3) et les secondes électrodes (4) sont agencées sur la couche de renfort (1).

7. Pansement de protection de plaie à micro-courant selon l'une quelconque des revendications 1 ou 4 à 6, dans lequel une taille de la couche de renfort (1) est plus grande qu'une taille de la couche adaptée à la peau (2), et une portion de la couche de renfort (1) au-delà de la couche adaptée à la peau (2) est munie circonférentiellement d'une couche adhésive (6).

8. Pansement de protection de plaie à micro-courant selon l'une quelconque des revendications 1 ou 4 à 6, dans lequel la couche adaptée à la peau (2) est constituée d'un matériau en gel ayant une propriété auto-adhésive.
